# EUROPÄISCHE PATENTSCHRIFT

(11) **EP 0 596 222 B1**
(45) Veröffentlichungstag und Bekanntmachung des Hinweises auf die Patenterteilung: **03.12.1997**
(21) Anmeldenummer: 93114654.2
(22) Anmeldetag: 13.09.1993
(51) Int. Cl.: G01N 33/86, G01N 21/03, G01N 11/14

(54) **Probenaufnahmeeinrichtung für die Blutgerinnungsmessung**
Sampling device for the measurement of blood coagulation
Dispositif d'échantillonage pour la mesure de la coagulation sanguine

(30) Priorität: 06.11.1992 DE 4237449
(43) Veröffentlichungstag der Anmeldung: 11.05.1994
(73) Patentinhaber: HEINRICH AMELUNG GmbH, D-32657 Lemgo (DE)
(72) Erfinder: Amelung, Rolf, Dipl.-Ing., D-32657 Lemgo (DE)
(74) Vertreter: Stracke, Alexander, Dipl.-Ing.

(56) Entgegenhaltungen:
- DE-B- 2 937 195

## Beschreibung

Die vorliegende Erfindung betrifft eine Probenaufnahmeeinrichtung für die Blutgerinnungsmessung, bestehend aus einer Meßküvette mit einem von ihrem Boden vorstehenden Zapfen und einer in der Meßküvette einliegenden Kugel.

Derartige Meßküvetten mit einliegender Kugel sind zum Einsatz in Coagulometern bekannt (DE 29 37 195 C3). Derartige Meßküvetten haben sich in der Praxis gut bewährt und dazu beigetragen, daß die sogenannte Kugelmethode sich bei den Blutgerinnungstests in den Coagulometern durchgesetzt hat. Es läßt sich nachweisen, daß sich bei Einsetzen der Gerinnung die aus dem Fibrinogengerinnungsansatz sich bildenden Fibrinfäden in erster Linie um die Kugeln und den Zapfgen wickeln, wobei daran zu denken ist, daß die Meßküvette im Coagulometer rotiert und damit auch die Kugel durch den Kontakt insbesondere zum Küvettenboden ebenfalls rotiert und sich die um die Kugeln wickelnden Fibrinfäden durch die Kugelrotation um den Zapfen geschlungen werden. Im Verhältnis zu den früher üblichen Meßküvetten ohne Zapfen ergibt sich somit eine deutlich exaktere und sichere Erfassung der Gerinnungszeit. Proben mit einem Fibrinogengehalt im Gerinnungsansatz von bis hin zu 90 mg/dl können noch hinreichend exakt bearbeitet werden.

Im Sinne des Erfordernisses der Kosteneinsparung im Gesundheitswesen gehen nunmehr neuerdings zahlreiche Anwender derartiger Coagulometer dazu über, nur noch die halbe Menge, manchmal noch weniger, des Reagenzes und des Plasmas für den Gerinnungsansatz zu nehmen. Dies bedeutet, daß dann im Gerinnungsansatz ein entsprechend verringerter Anteil von Fibrinogen enthalten ist, sich das Fibringerüst auch nur schwächer ausbildet und insbesondere dann Probleme auftreten können, wenn aufgrund des entsprechenden Krankheitsbildes des Patienten von vornherein schon ein erheblicher, im therapeutisch wichtigen Bereich liegender Fibrinogenmange vorliegt.

Der vorliegenden Erfindung liegt von daher die Aufgabe zugrunde, eine Probenaufnahmeneinrichtung der gattungsgemäßen Art zu schaffen, die auch noch bei einem gegenüber dem bisherigen stark verringerten Fibrinogenanteil im Gerinnungsansatz eine exakte und sichere Erfassung der Gerinnungszeit gewährleistet.

Die erfindungsgemäße Lösung besteht gemäß kennzeichnendem Teil des Patentanspruchs 1 darin, daß die kürzeste Entfernung zwischen der Kugel- und der Zapfenoberfläche 30 - 70 % des Kugeldurchmessers beträgt.

Die Entfernung in der Anfangsphase der Gerinnungsmessung zwischen der Kugel- und der Zapfenoberfläche ist damit gegenüber dem bisherigen deutlich vergrößert. Bislang wurde in der Anfangszeit der Gerinnungsmessung die Kugel in einem solchen Abstand zum Begrenzungszapfen gehalten, daß die kürzeste Entfernung zwischen der Kugel- und der Zapfenoberfläche nur 5 - 10% des Kugeldurchmessers betrug.

Es hat sich nun völlig überraschend gezeigt, daß durch diese Abstandsvergrößerung eine exakte Kugelmitnahme selbst bei stark verringertem Fibrinogengehalt im Gerinnungsansatz möglich ist. Es hat sich herausgestellt, daß beispielsweise noch bei einem Fibrinogengehalt von nur 50 mg/dl im Gerinnungsansatz eine zuverlässige exakte und zeitgerechte Kugelmitnahme gewährleistet ist. Bei entsprechenden Versuchsbedingungen kann auch mit noch geringerem Anteil gearbeitet werden. So hat sich beispielsweise herausgestellt, daß sich das Ergebnis im Sinne der Aufgabenstellung noch weiter verbessern läßt, wenn, gemäß einer bevorzugten Ausführungsform vorgesehen, der Boden der Meßküvette vom Ende der Seitenwandung in Richtung auf den Zapfen zu zumindest teilweise schräg abfallend ausgebildet ist.

Der nachweisliche, vorstehend geschilderte Effekt ist umso überraschender, als zunächst ja davon ausgegangen werden mußte, daß im Hinblick auf einen nur sehr geringen Fibrinogengehalt mit entsprechend schwachem Fibrinfadennetz in der Gerinnungsphase der relativ große Abstand zwischen der Kugel- und der Zapfenoberfläche eher bezuglich der Kugelmitnahme schädlich sein muß. Daß nun aber ganz im Gegenteil bei nur schwach sich ausbildendem Fibrinnetz eher eine exakte und zeitgerechte Kugelmitnahme erfolgt, liegt womöglich daran, daß in der Anfangsphase der Blutgerinnungsmessung und in der Anfangsphase der einsetzenden Gerinnung zwischen dem Zapfen und der Kugel nunmehr eine kleine Zone relativer Ruhe liegt, in der der Einfluß der sich drehenden Kugel schwach ist, die drehende Kugel also nicht immer wieder schädigend auf das sich bildende Fibrinnetz und insbesondere schädigend auf dessen Tendenz der Anhaftung am Zapfen einwirken kann, so daß sich zum gegebenen Zeitpunkt, wenn nämlich das Netz und deren Anhaftungstendenz auf die Kugel stark genug ist, die Kugel in Richtung auf den Zapfen zu in einer raschen Momentanbewegung und Überwindung der sie in der Anfangsphase auf Abstand haltenden Kräfte in ein trotz niedrigem Fibrinogenanteil relativ gut ausgebildetes Fibrinnetzwerk hineinbewegt und darin Halt findet. Dabei kann durch den gemäß einer bevorzugten Ausführungsform vorgesehenen zumindest teilweisen Schrägabfall des Meßküvettenbodens zum Zapfen hin diese Momentanbewegung der Kugel noch beachtlich begünstigt werden.

Man ist ferner bei der Messung der Blutgerinnung immer wieder mit dem unerklärlichen Phänomen konfrontiert, daß hin und wieder eine Vorgerinnung einsetzt, mit dem Ergebnis, daß der Gerinnungseintritt somit zu früh angezeigt wird. Diesem Störfaktor wirkt..der deutlich vergrößerte Abstand zwischen der Kugel-und der Zapfenoberfläche in der Anfangsphase der Blutgerinnungsmessung außerordentlich vorteilhaft entgegen, da normalerweise diese unerwünschte Vorgerinnung noch nicht ausreicht, den Raum zwischen Zapfen und Kugel so auszufüllen, daß es zu einer vorzeitigen Mitnahme der Kugel und Überwindung ihrer Haltekräfte kommt.

Unter Ausschaltung des vorstehend geschilderten bisherigen Störfaktors ermöglicht die erfindungsgemäße Ausgestaltung also die Bearbeitung von Proben mit einem gegenüber den bisherigen außerordentlich niedrigen Fibrinogengehalt, was es einerseits ermöglicht, mindestens mit halbem Testansatz im Verhältnis zum bisherigen Einsatz an Reagenz und Plasma zu arbeiten und andererseits auch patientenseitige Krankheitsfälle mit außerordentlich niedrigem Fibrinogenanteil im Blut noch exakt zu bearbeiten.

Ein Ausführungsbeispiel des Gegenstandes der Erfindung wird nachstehend unter Bezugnahme auf die Zeichnung näher beschrieben.
Es zeigen:
- Figur 1: einen Querschnitt durch eine Probenaufnahmeeinrichtung gemäß der Erfindung in mehrfach vergrößertem Maßstab,
- Figur 2: eine Querschnittsdarstellung einer weiteren Ausführungsform.

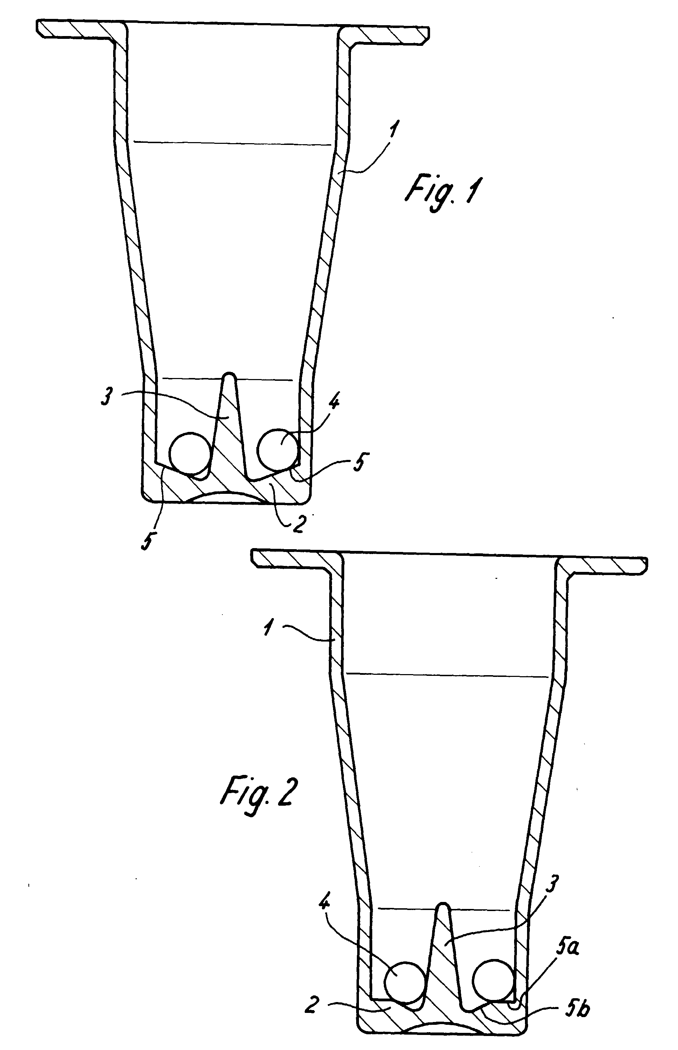

Die in Figur 1 dargestellte Probenaufnahmeeinrichtung für die Blutgerinnungsmessung besteht aus einer Meßküvette 1, die zweckmäßig die Form eines drehsymmetrischen kleinen Meßröhrchens aus Kunststoff hat. Von dem Boden 2 der Meßküvette 1 steht zentrisch ein Zapfen 3 vor. In der Meßküvette 1 liegt eine Kugel 4 ein. Im rechten Teil der Darstellung der Figur 1 ist illustriert, daß die Kugel beim Einsatz der Meßküvette in einem Coagulometer in der Anfangsphase der Blutgerinnungsmessung in einem Abstand mit dem Zapfen 3 gehalten ist. Die Abstandshaltung geschieht in der Praxis im Regelfall dadurch, daß die Meßküvette in die geräteseitige Aufnahme in leichter Schräglage eingesteckt wird, so daß schon aufgrund der Schwerkraft die Kugel 4 in der Ecke zwischen Boden und angrenzender Seitenwand zum Liegen kommt. Üblicherweise werden durch die exakte geometrische Vorgabe durch die Schräganordnung auch noch magnetische Haltekräfte auf eine beispielsweise aus Stahl ausgebildete Kugel 4 ausgeübt, um ihr eine definierte Haltekraft aufzuerlegen, die dann durch eine entsprechend exakte Kraft, die von dem sich bildenden Fibringerüst auf die Kugel im Sinne ihrer Mitnahme mit der Drehbewegung der Meßküvette 1 überwunden wird.

Es hat sich gezeigt, daß ein Anfangsabstand, der einer kürzesten Entfernung zwischen der Kugel- und der Zapfenoberfläche in der Größenordnung von 30 - 70 % des Kugeldurchmessers entspricht, zu exakten Meßwerten bei sehr geringem Fibrinogengehalt im Probenansatz führt. Es können problemlos Proben mit 50 mg/dl und weniger Fibrinogen bearbeitet werden. Zunächst dreht sich die Kugel 4 durch die Kontaktnahme mit der Meßküvette, insbesondere mit deren Boden 2 um sich selbst. Es wickeln sich dabei die sich bildenden Fibrinfäden auch um die Kugel 4 und werden durch deren Bewegung auch um den Zapfen 3 geschlungen. Bei entsprechend ausgebildetem Fibrinnetz vollführt die Kugel 4 eine Bewegung auf den Zapfen 3 zu, bis hin gegebenenfalls zur Anlage am Zapfen 3 und wird im übrigen von der Drehbewegung der Meßküvette unter Überwindung der Haltekräfte, üblicherweise gebildet aus Schwerkraft und Magnetkraft, mitgenommen und in der üblichen Weise an bzw. von einem Sonsor vorbei- bzw. weggeführt, was zur Zeitmessung benutzt wird.

Die genannte Wanderbewegung der Kugel 4 in das Fibrinnetz hinein und auf den Zapfen 3 zu wird gemäß einer bevorzugten Ausführungsform dadurch begünstigt, daß der Boden 2 ausgehend von dem unteren Ende der Seitenwand der Meßküvette 1 zum Zapfen 3 hin schräg abfallend ausgebildet ist. Hierzu ist in der Ausführungsform nach Figur 1 die gesamte Bodenfläche als entsprechend schräg abfallender Konus 5 ausgebildet.

Im dargestellten Ausführungsbeispiel nach den Figuren ist bei der Abstandsbemessung von einem Beispiel ausgegangen, bei dem die kürzeste Entfernung zwischen der Kugel- und der Zapfenoberfläche 50 % des Kugeldurchmessers beträgt.
Zur Illustration des vorstehend Gesagten ist ferner jeweils auf der linken Hälfte der Figuren 1 und 2 die Kugel 4 anliegend an den Zapfen 3 dargestellt, also in ihrer möglichen Endlage, in der sie von der sich drehenden Meßküvette 1 unter Überwindung der Haltekräfte mitgenommen wird.

Zur Illustration der Größenverhältnisse sei darauf hingewiesen, daß sich für eine Einrichtung gemäß der Erfindung ein Kugeldurchmesser von ca. 2 mm als zweckmäßig erwiesen hat.

Das in Figur 2 dargestellte Ausführungsbeispiel unterscheidet sich von demjenigen nach Figur 1 dadurch, daß am Boden 2 der Meßküvette 1 angrenzend an das untere Ende der Seitenwand der Meßküvette 1 zunächst ein ebener Bodenbereich 5a vorgesehen ist, an den sich dann ein schräg zum Zapfen hin abfallender Abschnitt 5b anschließt. Der ebene Bodenabschnitt 5a kann dabei einer unerwünscht vorzeitigen Mitnahme der Kugel 4 entgegenwirken, während der schräge Abschnitt 5b die Bewegung der Kugel 4 auf den Zapfen 3 zu zum gewünschten Zeitpunkt erleichtert.

## Patentansprüche

1. Probenaufnahmeeinrichtung für die Blutgerinnungsmessung, bestehend aus einer Meßküvette (1) mit einem von ihrem Boden (2) vorstehenden Zapfen (3) und einer in der Meßküvette einliegenden Kugel (4), die in der Anfangsphase der Blutgerinnungsmessung in einem Abstand von dem Zapfen (3) gehalten ist, **dadurch gekennzeichnet**, daß die kürzeste Entfernung zwischen der Kugel- und der Zapfenoberfläche 30 - 70 % des Kugeldurchmessers beträgt.

2. Probenaufnahmeeinrichtung nach Anspruch 1, dadurch gekennzeichnet, daß der Boden (2) der Meßküvette (1) vom Ende der Seitenwand in Richtung auf den Zapfen (3) zu zumindest teilweise schräg abfallend (5, 5b) ausgebildet ist.

3. Probenaufnahmeeinrichtung nach Anspruch 2, dadurch gekennzeichnet, daß der Boden (2) angrenzend an die Seitenwand der Meßküvette (1) zunächst einen ebenen Abschnitt (5a) aufweist, an den sich zum Zapfen hin ein schräg abfallender Abschnitt (5b) anschließt.

4. Probenaufnahmeeinrichtung nach einem der vorhergehenden Ansprüche, dadurch gekennzeichnet, daß die Entfernung zwischen der Kugel- und der Zapfenoberfläche 50 % des Kugeldurchmessers beträgt.

5. Probenaufnahmeeinrichtung nach einem der vorhergehenden Ansprüche, dadurch gekennzeichnet, daß die Kugeloberfläche durch eine Schräglage der Meßküvette (1) in ihrer Entfernung zu der Zapfenoberfläche gehalten ist.

6. Probenaufnahmeeinrichtung nach einem der vorhergehenden Ansprüche, dadurch gekennzeichnet, daß die Kugel (4) aus Metall besteht und durch Magnetkraft in ihrem Abstand zu dem Zapfen (3) gehalten ist.

## Claims

1. A sample receiving device for blood coagulation measurement, comprising a measuring vessel (1) having a spigot (3) which projects from its bottom (2) and a ball (4) which is disposed in the measuring vessel and which is held at a spacing from the spigot (3) in the initial phase of the blood coagulation measurement procedure, characterised in that the shortest distance between the ball surface and the spigot surface is 30 - 70% of the ball diameter.

2. A sample receiving device according to claim 1 characterised in that the bottom (2) of the measuring vessel (1) is of a configuration such as to at least in part inclinedly fall away (5, 5b) from the end of the side wall towards the spigot (3).

3. A sample receiving device according to claim 2 characterised in that adjoining the side wall of the measuring vessel (1) the bottom (2) firstly has a flat portion (5a) which is adjoined towards the spigot by a portion (5b) which inclinedly falls away.

4. A sample receiving device according to one of the preceding claims characterised in that the distance between the ball surface and the spigot surface is 50% of the ball diameter.

5. A sample receiving device according to one of the preceding claims characterised in that the ball surface is held at its distance relative to the spigot surface by an inclined position of the measuring vessel (1).

6. A sample receiving device according to one of the preceding claims characterised in that the ball (4) comprises metal and is held at its spacing relative to the spigot (3) by magnetic force.

## Revendications

1. Dispositif d'échantillonnage servant à mesurer la coagulation sanguine, constitué d'une cuvette de mesure (1) comportant un téton (3) dépassant de son fond (2) et une bille (4) reposant dans la cuvette de mesure, qui est maintenue à distance du téton (3), dans la phase initiale de mesure de la coagulation sanguine, caractérisé en ce que la distance la plus courte entre la surface de la bille et la surface du téton représente de 30 à 70 % du diamètre de la bille.

2. Dispositif d'échantillonnage selon la revendication 1, caractérisé en ce que le fond (2) de la cuvette de mesure (1), à partir de l'extrémité de la paroi latérale, en direction du téton (3), présente une conformation (5a, 5b) au moins partiellement inclinée de façon oblique.

3. Dispositif d'échantillonnage selon la revendication 2, caractérisé en ce que le fond (2), de façon adjacente à la paroi latérale de la cuvette de mesure (1), présente tout d'abord un tronçon plan (5a), auquel fait suite un tronçon (5b) descendant en oblique en direction du téton.

4. Dispositif d'échantillonnage selon l'une des revendications précédentes, caractérisé en ce que la distance entre la surface de la bille et la surface du téton représente 50 % du diamètre de la bille.

5. Dispositif d'échantillonnage selon l'une des revendications précédentes, caractérisé en ce que la surface de la bille, du fait d'une inclinaison de la cuvette de mesure (1), est maintenue à distance de la surface du téton.

6. Dispositif d'échantillonnage selon l'une des revendications précédentes, caractérisé en ce que la bille (4) est constituée en métal et est maintenue écartée du téton (3) par une force magnétique.
